# EUROPEAN PATENT SPECIFICATION

(11) **EP 0 762 121 B1**
(45) Date of publication and mention of the grant of the patent: **18.10.2000**
(21) Application number: 96112800.6
(22) Date of filing: 08.08.1996
(51) Int. Cl.: G01N 33/53

(54) **Antigen for enzyme immunoassay and method of measuring anti-erythrocyte antibody**
Antigen für Enzymimmuntest und Verfahren zur Messung von erythrozytären Antikörpern
Antigène pour immunoessai enzymatique et méthode pour mesurer des anticorps d'érythrocyte

(30) Priority: 09.08.1995 JP 22578495
(43) Date of publication of application: 12.03.1997
(73) Proprietor: NIPPON ZOKI PHARMACEUTICAL CO., LTD., Chuo-ku, Osaka (JP)
(72) Inventor: Yoshii, Haruo, c/o Inst. of Bio-Active Science, Yashiro-cho, Katoh-gun, Hyogo (JP); Fukata, Yuriko, c/o Inst. of Bio-Active Science, Yashiro-cho, Katoh-gun, Hyogo (JP)
(74) Representative: Hansen, Bernd, Dr. Dipl.-Chem.

(56) References cited:
- US-A- 5 179 198
- US-A- 5 288 610
- ANAL. CHEM. (ANCHAM);77; VOL.49 (13); PP.2083-6, STATE UNIV. NEW YORK;DEP. CHEM.; BUFFALO; N. Y., XP002019048 D'ORAZIO P ET AL: "Ion electrode measurements of complement and antibody levels using marker-loaded sheep red blood cell ghosts"
- DATABASE WPI Section Ch, Derwent Publications Ltd., London, GB; Class B04, AN 84-058770 XP002019049 "Aq. Medium for Agglutination Test" & JP-A-59 015 859 ((GREC) GREEN CROSS CORP.) , 26 January 1984

## Description

### Field of the Invention:

The present invention relates to a novel enzyme immunoassay for anti-erythrocyte antibody utilizing an antigen consisting of membrane fragments of erythrocytes.

### Background of the Invention:

As one of the test systems for developing the drugs which controls the immune system such as immunosuppressive agents and immunopotentiators and also for checking the changes of immune system, a test method using an antibody production of animals as an index has been used frequently. There is a method in which erythrocytes are used as antigen for inducing the antibody production and erythrocytes of horse and sheep have been sold as a reagent since they are available in large quantities. Among them, sheep erythrocyte (sheep red blood cell: SRBC) is most widely used as antigenic erythrocyte especially because of its easy storage and handling. In "Seikagaku Jiten (Dictionary of Biochemistry)" (second edition; published by Tokyo Kagaku Dojin), SRBC is explained as an independent lemma whereby it is apparent that SRBC has been widely used.

SRBC as an antigen can easily induce the antibody production and the cellular immunity by its sole administration without mixing with adjuvants (immunopotentiating substances) and, accordingly, it has been widely used, for example, in immunopharmacological and immunotoxicological studies. As a method of measuring anti-SRBC antibody in blood, a classic method utilizing agglutination is used mostly as a practical measuring method. However, said method has a disadvantage that it is discontinuous and is poor in terms of fine quantitativeness and, in addition, it has another disadvantage that the agglutination is confirmed by naked eye whereby there is no objectivity. Accordingly, various attempts have been made for an objective and more quantitative measuring method, i.e. a method of measuring the anti-SRBC antibody by means of enzyme immunoassay (ELISA). However, the methods up to now still remain some problems to be solved and are hardly said to be accurate, easy and convenient.

For example, in a method by Mori et al. [Int. J. Immunopharmac., Vol. 11, 597-606 (1989)] SRBC per se is used as a granule antigen and is directly insolubilized on a plate by a special means. However, turbidity is poor and, moreover, red color of erythrocytes remains in an insolubilized antigen. Therefore, said method is not suitable for ELISA wherein the reaction product is measured by means of absorbance using marker enzyme and, in addition, there is a problem in its reproducibility.

There are other methods such as a method by Temple et al. [Fundam. Appl. Toxicol., 21, 412-419 (1993)] and that by Van Loveren et al. [Int. J. Immunopharmac., 13, 689-695 (1991)] in which membrane protein of SRBC is solubilized under a specific condition and said soluble protein is used as an antigen for insolubilization. In a method by Van Loveren et al, membrane protein is solubilized by treating with potassium chloride of a high concentration. The substance which is used in those methods is thought to be a part of membrane protein which is solubilized and a problem still remains with respect to the difference in antigenicity between such partial membrane protein and the original SRBC and, in addition, the solubilizing and separating operations require time and trouble. As such, no suitable insolubilize antigen is used in the methods which have been reported up to now and various problems have been left remained for their practical application.

US-A-5,179,198 describes an isolated human erythrocyte cell membrane fraction. The procedure is such that it will be free of soluble cytoplasm proteins and haemoglobin.

Anal. Chem. Vol. 49 (1977) discloses a method for producing sheep erythrocyte "ghosts", which are free of soluble cytoplasm proteins and haemoglobin.

JP-A-59015859 relates to an improved agglutination assay, by using an antibody sensitizing sheep erythrocyte used as flocculation test carrier. Erythrocyte membrane components are used as a material for preventing non-peculiar agglutination reaction, i.e. the erythrocyte membrane components can enhance the sensitivity of the antibody sensitizing sheep erythrocyte agglutination.

US-A-5,288,610 concerns a detecting agent for an anti-platelet antibody comprising - among other components - a platelet antigen component immobilized on the surface of a carrier particle.

### Detailed Description of the Invention:

The object of the present invention is to offer a novel enzyme immunoassay for anti-erythrocyte antibody characterized in using an antigen containing membrane fragments of erythrocyes. The present invention discloses an enzyme immunoassay wherein said antigen essentially consists of cell membrane fragments which are obtained by destruction of erythrocytes so as not to contain soluble cell cytoplasm protein such as hemoglobin. In this context, "essentially consisting" means that the antigen may contain inevitable impurities, such as proteins or other compounds, which however do not deteriorate its antigenic properties, in particular do not disturb an enzyme immunoassay using said antigen. In one embodiment said antigen is contained in a composition comprising further components or carriers as conventionally used in this field, such as water, buffers, salts, preservatives and so on. This assay is practical and has a good quantitativeness whereby the above-mentioned problems in the conventional methods are solved.

The characteristic feature of the present invention is to use fragments of cell membrane of erythrocytes as antigen for insolubilization where said cell membrane fragments of erythrocytes are the residual membrane fraction after removing the soluble cytoplasm proteins such as hemoglobin which interfere with the absorbance analysis of ELISA. The cell membrane fragments of erythrocytes wherefrom cytoplasm proteins such as hemoglobin are removed can be prepared by destructing the erythrocytes followed by subjecting the soluble cytoplasm proteins and membrane components to a fractionation. In destructing the erythrocytess, known means such as hemolytic destruction, freezing-and-thawing and ultrasonic treatment may be applied. For example, the hemolytic destruction can be easily conducted by a simple operation of exposing the erythrocytes to a hypotonic solution such as water and is easy to apply.

Then, with respect to a method of separating soluble cytoplasm protein such as hemoglobin and cell membrane fragments, known means such as gel filtration, ultrafiltration and centrifugation may be applied. For example, in the case of gel filtration, it is enough to roughly separate into two fractions - cell membrane fragments having high molecular weight and other soluble cytoplasm proteins having low molecular weight - and, when a carrier exhibiting a suitable fractionating coverage is used, only cell membrane fragments can be eluted to a void volume part and can be easily separated from soluble cytoplasm protein fractions with low molecular weights which are eluted later whereby the antigen of the present invention is able to be prepared by simple operations.

The antigen used in the present invention (cell membrane fragments of erythrocytes) obtained by separating from the soluble cytoplasm protein fractions of erythrocytes as such is insolubilized in a plate for enzyme immunoassay whereupon a conventional ELISA can be carried out. With respect to the technique for ELISA such as insolubilizing method of antigen, blocking method, kind of enzyme marking, selection of the substrates and the conditions for enzymatic reaction and final measuring method, conventional known art may be utilized. Such techniques are mentioned in detail, for example, in "Koso Men-eki Sokuteiho (Enzyme Immunoassay)" (third edition; edited by Ishikawa, Kawai and Miyai; published by Igaku Shoin) and other references.

Erythrocytes from any species of animal can be applicable to prepare the antigen of the present invention, and it may be possible to choose the erythrocyte species according to its availability, antigenecity and the animal species used in the test for antibody production, etc. Usually, SRBC which has been widely used in this field is usable.

### Brief Explanation of the Drawings:

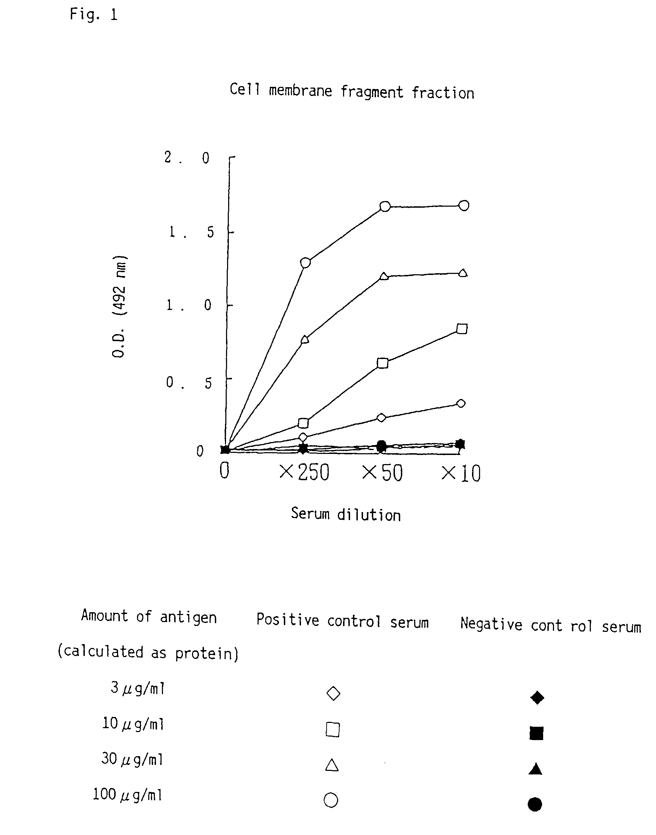

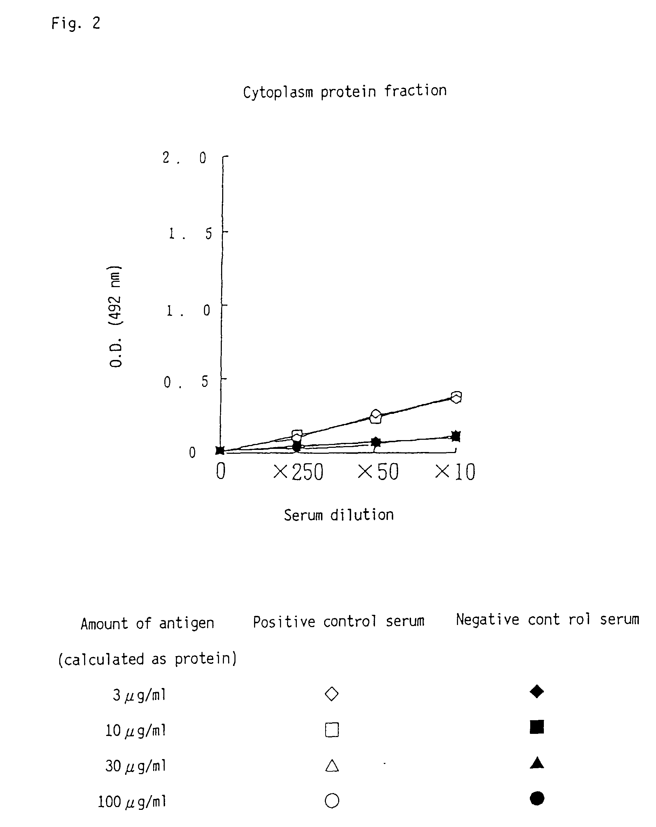

An example of the result of ELISA which was conducted using the antigen of the present invention consisting of erythrocyte cell membrane fragments is shown in Fig. 1; and the result when cytoplasm protein fractions of erythrocytes was used as an immobilized antigen is shown in Fig. 2.

An embodiment of the present invention will be illustrated by way of the following examples.

### Examples:

### Referential Example 1. Preparation of Positive Control Serum and Negative Control Serum

SRBC (0.2 ml) wherein the concentration was adjusted to 5 x 109 erythrocytes/ml using a physiological saline solution was administered to BALB/c mice intraperitoneally and serum on the fifth and sixth days was pooled to give a positive control serum while the serum of normal BALB/c mice was pooled to give a negative control serum.

### Example 1. Fractionation of SRBC Membrane Fragments by Gel Filtration

To 1.0 ml of pellets of SRBC was added the same amount (1.0 ml) of distilled water to hemolyze, a part of it was applied to a column of Sepharose CL-6B (manufactured by Pharmacia; 25 mm x 69 mm) equilibrated with 0.1 M carbonate buffer (pH 9.5) and a gel filtration was conducted under the fractionating condition of 3.0 ml/tube at 4°C. Eluted fractions were measured at the absorbance of 280 nm whereupon two peaks - a small peak (fractions 28-31) corresponding to void volume and a peak (fractions 56-67) presumably containing soluble cytoplasm protein coinciding with the peak of hemoglobin - were noted. Then the peak of the void volume part and the peak eluted thereafter were pooled as a cell membrane fragment fraction and a cytoplasm protein fraction, respectively and used as insolubilized antigen in the ELISA.

### Example 2. Measurement of Anti-SRBC Antibody in ELISA.

Both fractions obtained in Example 1 were diluted with a 0.1 M carbonate buffer (pH 9.5) to adjust the concentration as protein to 3-100µg/ml (based upon the absorbance at 280 nm). Each of the fraction solutions (100µl) was added to each well of the plate for ELISA having 96 wells and insolubilized at 4°C overnight. Then 200µl of a phosphate buffer containing 0.1% gelatin (G-PBS) was added thereto followed by blocking at room temperature for one hour. This was washed three times with a phosphate buffer containing 0.05% of Tween 20 (T-PBS), then 100µl of the positive or negative control serum diluted with G-PBS was added to each of the wells and a reaction was conducted at room temperature for two hours. This was washed three times with T-PBS, then 100µl of anti-mouse IgG marked with horse radish peroxidase diluted to an extent of 500-fold with G-PBS was added thereto and the reaction was conducted at room temperature for two hours. This was again washed with T-PBS three times, then 100µl of a coloring solution (prepared by adding 10 mg of o-phenylenediamine dihydrochloride and 10µl of 33% hydrogen peroxide to 10 ml of a citrate buffer [pH 4.5]) was added and the reaction was conducted at room temperature for 5-10 minutes. The reaction was stopped with 100µl of 1 N hydrochloric acid and the absorbance at 492 nm was measured. An example of the result of the ELISA using the cell membrane fragment fraction as an antigen for insolubilization is given in Fig. 1 while the result of the case where the cytoplasm protein fraction was used as an antigen for insolubilization is given in Fig. 2.

As shown in Fig. 1 and Fig. 2, when anti-SRBC-IgG antibody in the positive control serum wherein antibody production was induced was used, the absorbance increased depending upon the antigen concentration in the measuring system of the present invention using the cell membrane fragment fraction of erythrocytes as an insolubilized antigen in ELISA while, in the system where cytoplasm protein fraction is used as an insolubilized antigen, there was no concentration-dependency at all and the anti-SRBC-IgG antibody was not measured quantitatively. Needless to say, antibody was not measured in the serum in the negative control serum which was serum of normal mice.

Sepharose CL-6B was used as a carrier in a fractionation by gel filtration in Example 1 and this carrier for the gel filtration is a globular protein and its fractionating range in terms of molecular weight is from 10,000 to 4,000,000. Accordingly, molecular weight of the membrane fragment obtained by destruction of erythrocytes is supposed to be more than 4,000,000. However, it is principally possible to utilize any kind of carrier provided that such a carrier is capable of separating erythrocyte membrane fragment and cytoplasm proteins and also to utilize any other method such as ultrafiltration and centrifugation.

In ELISA, turbidity and degree of coloration of the insolubized antigen have a big influence on the accuracy of the measurement. A solution of cell membrane fragments of erythrocytes which is an antigen of the present invention is almost transparent and is very useful as an insolubilized antigen for ELISA. Since the antigen of the present invention can be prepared by very simple operations as mentioned above, it is possible to give large quantities of antigen for insolubilization at one time. In addition, the resulting antigen of the present invention consisting of membrane fragments of erythrocytes can be stored in cool (e.g. 4°C) and dark place after preparing into a solution in a phosphate buffer and, even when stored for ten months for example, measurement is still possible in the same manner as in the prepared stage and, therefore, the antigen of the present invention consisting of the erythrocyte membrane fragment and the measuring method using it may be said to be very practical.

Further, when compared with a method mentioned in the paragraph of the prior art wherein a part of solubilized membrane protein of erythrocytes is used, the measuring method utilizing the antigen of the present invention is able to measure the amount of antibody more accurately from immunological viewpoint as well.

As mentioned hereinabove, the present invention is with simple operations and is practicable without too much time and cost and it is now possible to conduct an enzyme immunoassay of anti-erythrocyte antibody having accuracy, high sensitivity and excellent quantitativeness.

## Claims

1. A method for measuring anti-erythrocyte antibody in an enzyme immunoassay wherein an antigen for an enzyme immunoassay essentially consisting of cell membrane fragments of erythrocytes which are essentially free of soluble cytoplasm proteins, is used.

2. Method according to claim 1, wherein said cell membrane fragments are essentially free of haemoglobin.

3. Method according to claim 1 or 2 wherein said cell membrane fragments are obtained from SRBC (sheep red blood cells).

## Patentansprüche

1. Verfahren zur Messung von Anti-Erythrozyten-Antikörper in einem Enzym-Immunosassay, worin ein Antigen für einen Enzym-Immunoassay, bestehend im wesentlichen aus Zellmembranfragmenten von Erythrozyten, die im wesentlichen frei sind von löslichem Cytoplasmaproteinen, verwendet wird.

2. Verfahren gemäß Anspruch 1, worin die Zellmembranfragmente im wesentlichen frei von Hämoglobin sind.

3. Verfahren gemäß Anspruch 1 oder 2, worin die Zellmembranfragmente aus SRBC (rote Blutzellen vom Schaf) erhalten werden.

## Revendications

1. Procédé de dosage d'anticorps anti-érythrocytes par une analyse immuno-enzymatique, dans lequel on utilise pour l'analyse immuno-enzymatique un antigène constitué essentiellement de fragments de membrane cellulaire d'érythrocytes qui sont essentiellement dépourvus de protéines cytoplasmiques solubles.

2. Procédé selon la revendication 1, dans lequel lesdits fragments de membrane cellulaire sont essentiellement dépourvus d'hémoglobine.

3. Procédé selon la revendication 1 ou 2, dans lequel lesdits fragments de membrane cellulaire sont obtenus à partir de SRBC (globules rouges de mouton).
